(19) Europäisches Patentamt
European Patent Office
Office européen des brevets

(11) Numéro de publication: **0 318 398 B1**

(12) # FASCICULE DE BREVET EUROPEEN

(45) Date de publication de fascicule du brevet: **15.03.95**

(51) Int. Cl.[6]: **A61K 9/54**, A61K 31/55

(21) Numéro de dépôt: **88402981.0**

(22) Date de dépôt: **25.11.88**

(54) **Forme à libération prolongée du diltiazem et son médicament ainsi obtenu.**

(30) Priorité: **26.11.87 FR 8716425**

(43) Date de publication de la demande:
**31.05.89 Bulletin 89/22**

(45) Mention de la délivrance du brevet:
**15.03.95 Bulletin 95/11**

(84) Etats contractants désignés:
**BE CH DE ES FR GB GR IT LI LU NL SE**

(56) Documents cités:
**EP-A- 0 077 956**
**EP-A- 0 149 920**
**EP-A- 0 154 895**
**GB-A- 2 167 374**

(73) Titulaire: **ETHYPHARM**
**21, rue Saint-Matthieu**
**F-78550 Houdan (FR)**

(72) Inventeur: **Boyer, Jean-François**
**73, rue des Jeux de Billes**
**F-78550 Houdan (FR)**
Inventeur: **Debregeas, Patrice**
**10, rue du Peintre Lebrun**
**F-78000 Versailles (FR)**
Inventeur: **Leduc, Gérard**
**La Grange La Brosse**
**F-45330 Malesherbes (FR)**

(74) Mandataire: **Pinguet, André**
**Cabinet de Proprieté Industrielle**
**CAPRI,**
**94, avenue Mozart**
**F-75016 Paris (FR)**

**EP 0 318 398 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une nouvelle forme galénique du Diltiazem chlorhydrate (désigné ci-après plus simplement Diltiazem), et son procédé de préparation.

Ce corps a pour formule :

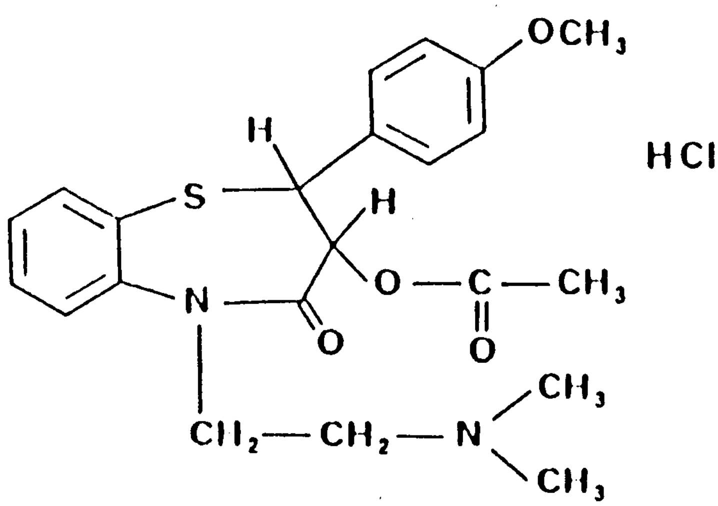

Il se présente sous la forme d'une poudre ou de cristaux blancs, inodores, ayant un point de fusion compris entre 208 et 212 °C. Le Diltiazem est connu comme inhibiteur de l'influx calcique à travers les canaux spécifiques membranaires d'une variété de cellules. Par ce mécanisme, il exerce un effet dépresseur sur l'activité pacemaker cardiaque et sur la conduction nodale auriculo-ventriculaire résultant en un effet chronotrope négatif. Le Diltiazem semble avoir un faible effet inotrope negatif sur le muscle cardiaque et entraîne une vasodilatation des muscles lisses. Le Diltiazem réduit le sodium intracellulaire par stimulation de la pompe sodium-potassium des muscles lisses et d'autres types de cellules. Cet effet est responsable des effets stabilisateurs de membrane et de l'action diurétique (non vasculaire) sur les tubules rénaux. Cliniquement, le Diltiazem est surtout utilisé comme anti-angineux, aussi bien dans l'angor chronique stable que dans l'angor instable. Il possède également une activité dans les arythmies et peut être utilisé dans d'autres affections cardiovasculaires telles que l'insuffisance cardiaque congestive et le syndrome de Raynaud, ainsi que dans l'insuffisance circulatoire cérébrale et périphérique.

Cependant, le Diltiazem est actuellement très étudié dans l'hypertension, indication déjà reconnue par les autorités de nombreux pays.

C'est un produit qui possède peu d'effets secondaires, surtout si on le compare à d'autres antagonistes du calcium comme la Nifédipine ou le Vérapamil, et est donc envisageable dans des traitements à long terme.

Le Diltiazem est utilisé actuellement sous forme de comprimés à libération immédiate, dosés à 30, 60 et 90 mm. La posologie est de 180 mg répartis en deux ou trois prises et, dans les cas sévères 240 mg, voire 360 mg, par jour.

L'étude bibliographique montre que le Diltiazem a une absorption très rapide et que le pic de concentration est atteint entre trois heures un quart et quatre heures. La demi-vie est de quatre à sept heures. On n'observe pas de phénomène de saturation aux doses thérapeutiques et, après administrations répétées, par conséquent, il n'y a pas accumulation du médicament. La tolérance du Diltiazem est très bonne. Une variabilité importante a été observée entre les paramètres pharmacocinétiques des sujets, ce qui rend difficile le maintien des concentrations plasmatiques dans la fenêtre thérapeutique. Ces différentes données et l'expérience acquise lors de la mise au point de nombreux produits ont incité la demanderesse à tenter d'optimiser la forme galénique de Diltiazem déjà existante en choisissant une forme a libération prolongée présentée en gélules en comprimés ou en sachets contenant des microgranules.

La forme à libération prolongée doit apporter une réduction du nombre de prises journalières en prolongeant la libération du produit selon une prise si possible, deux au maximum. Ainsi, pour améliorer le confort du patient et éviter d'une part un trop grand nombre de prises dans la journee (risque de non respect de la posologie) et d'autre part, réduire le risque d'effets indésirables liés à des pics sanguins très élevés, on a cherché, conformément à la présente invention, à maintenir le taux sanguin à l'état d'équilibre

au minimum a 40 ng/ml, et avec un maximum ne dépassant pas 300 mg/ml. Un tel resultat n'est pas possible avec les formes usuelles ou du type comprimés à action immédiate ou comprimés à action retard tels que commercialisés en Europe, en une ou deux prises par jour, car elles ne permettent pas de rester dans les limites de taux sanguins ci-dessus De plus, la forme microgranules choisie permet une meilleure dispersion du principe actif. Après l'ouverture de la gélule, les microgranules se repandent largement dans le tractus digestif entraînant une augmentation de la surface de contact principe actif - liquides digestifs, et permettent ainsi une bonne distribution de l'effet médicamenteux par une meilleure absorption. D'autre part, le fractionnement de l'unité de dosage rend impossible toute création d'une haute concentration de substances actives en un point du tractus digestif, diminuant ainsi les intolérances locales et les variations intra et inter patients.

On connait des tentatives de réalisation de microgranules dans l'art antérieur contenant du Diltiazem. Ainsi, la demande de brevet europeen n° 0 149 920, déposée au nom de ELAN CORPORATION plc, décrit des microgranules contenant du Diltiazem en association avec un acide organique et un lubrifiant dans la membrane extérieure (selon la pharmacopee américaine n° 21 - "United States Pharmacopea No 21") qui ne permettent pas d'obtenir des taux sanguins circulants suffisants et au minimum de 40 ng/ml en une ou deux prises par jour pour des doses journalières comprises entre 100 et 500 mg, de préférence 150 à 400 mg sans apparition d'effets secondaires néfastes. En outre, la stabilité de tels microgranules peut être affectée par la présence des acides organiques qui peuvent influer sur les caractéristiques physico-chimiques des membranes d'enrobage extérieures ou intérieures des microgranules.

L'invention a pour objet une forme galénique à libération prolongée de Diltiazem pharmaceutiquement acceptable comportant des microgranules, du type constitué en son centre d'un noyau garni de couches comportant le principe actif, chaque microgranule comportant une membrane externe, caractérisée en ce que celle-ci est adaptée pour libérer le Diltiazem appliqué sur le noyau neutre dans un milieu aqueux selon des proportions suivantes, mesurées selon la méthode de la pharmacopée américaine USP n° 21

a) entre 5 et 35 % après une heure
b) entre 15 et 40 % après deux heures
c) entre 20 et 50 % après trois heures
d) entre 30 et 75 % après quatre heures
e) entre 40 et 80 % après six heures
d) entre 55 et 95 % après huit heures

et en ce qu'elle comporte, exprimé en poids total de micro-granules :

| | |
|---|---|
| - noyau neutre → | 8 à 15 % |
| - couche active constituée de | |
| Diltiazem →<br>Polyvinylpyrrolidone →<br>- membrane externe → | 60 à 85 %<br>4 à 6 %<br>1 à 20 % |

Le noyau aura de 0,2 à 0,5 mm de diamètre et sera constitué d'excipients neutres, par exemple d'environ 75 % de saccharose ou de fructose et de 25 % d'amidon.

Conformément à l'une des variantes du procédé selon l'invention, on introduit les microgranules neutres dans une turbine de dragéification et dont la taille est comprise de préférence entre 0,2 et 2 mm.

On prépare une solution de PVP (polyvinylpyrrolidone) dans l'eau ou un solvant organique solubilisant (par exemple alcool éthylique, acétone), et on mouille les microgranules avec la solution de PVP et on projette une quantité de principe actif jusqu'à séchage des granules. On renouvelle l'opération jusqu'à épuisement de la quantité de principe actif correspondant au dosage.

Ensuite, on sèche les microgranules à l'air chaud et/ou par passage dans une enceinte de dessiccation. On tamise et on contrôle l'humidité et la granulométrie.

Conformément à l'invention, plusieurs types d'enrobage permettent de realiser la membrane externe. Selon l'invention, ces enrobages seront considérés comme équivalents si ils permettent d'obtenir des produits bio-équivalents à ceux décrits ci-après à titre d'exemple non limitatifs, les résultats de bio-disponibilité étant indiqués ci-après pour les produits correspondant à ces exemples afin de permettre à l'homme de l'art d'apprécier ladite bio-équivalence.

Dans une première série d'exemples, on a utilisé comme constituant principal de la membrane externe de la forme galénique, un mélange de gomme laque et d'éthylcellulose.

Dans ce cas, on prépare pour l'enrobage une solution alcoolique de gomme laque, et d'éthylcellulose ainsi que la quantité appropriée de talc.

On applique la solution alcoolique de gomme laque décirée et d'éthylcellulose et on projette simultanément le talc. On sèche à l'air chaud et on tamise, on contrôle l'humidité et le granulométrie.

Après acceptation du produit, suivant les contrôles prévus en cours de fabrication, on procède à la mise en gélules des microgranules ou à la fabrication de comprimés contenant ces microgranules.

Les caractéristiques de la membrane externe des microgranules ainsi obtenues doivent permettre de libérer le Diltiazem en milieu aqueux selon la méthode américaine USP n° 21 dans les intervalles suivants:

10 à 20 % au bout d'une heure
30 à 45 % au bout de quatre heures
60 à 75 % au bout de huit heures.

Pour cela, on a utilisé une solution alcoolique de gomme laque décirée et d'éthylcellulose dont les proportions respectives exprimées en poids sec sont de 70 parties en poids de gomme laque pour 30 parties en poids d'éthylcellulose.

Selon les formes de réalisation des microgranules dont la couche externe contient la gomme laque, les limites de chaque composant utilisé figurent dans le tableau 1, exprimées en proportion en poids par rapport au poids total de produit fini (microgranules en vrac).

TABLEAU 1

| COMPOSANTS | PROPORTION % |
|---|---|
| Granule neutre | 8-15 |
| Diltiazem | 60-85 |
| Polyvinylpyrrolidone | 4-6 |
| Gomme laque | 2-5 |
| Ethylcellulose | 1-3 |
| Talc | 0-10 |

Pour mieux faire comprendre l'invention, on a indiqué sur la figure 1 et les courbes de délitement in vitro (méthode américaine USP n° 21) limites correspondant au cas de la couche externe comprenant la gomme laque (courbes n° 1 et n° 2) ainsi que deux exemples de formulation, la première (courbe n° 3) qui comporte 3,50 % en poids sec de gomme laque et d'éthylcellulose et 6,95 % en poids de talc et qui est une formulation dont les normes ne correspondent pas à des microgranules conformes à l'invention, et la seconde (courbe n° 4) qui comprend 4,75 % de gomme laque et d'éthylcellulose et 9,90 % en poids de talc, dont les caractéristiques correspondent à une formulation conforme à la présente invention.

On a par ailleurs fait figurer les résultats obtenus en biodisponibilité en utilisant la formulation correspondant à la courbe n° 4 de la figure 1. Ces résultats sont reportés dans le tableau II ci-après pour trois dosages, par prise (180, 240, et 300 mg de Diltiazem par gélule) qui sont les trois dosages retenus comme intéressants après réalisation d'une étude d'intervalle de dose efficace réalisée par la demanderesse. Ces études de biodisponibilité du tableau II ont été réalisées sur vingt-quatre volontaires en étude croisée, et en prises répétées en comparant le produit selon l'invention (référence SR) par rapport au produit du commerce commercialisé sous la marque TILDIEM® de la société SYNTHELABO (comprimé dosé à 60 mg de Diltiazem). Excepté aux jours 1 et 7 de l'étude, les patients ont reçu l'équivalent de 180 mg à 240 mg par jour pour les études testant ces dosages. Dans l'étude testant le 300 mg SR, le dosage du produit immédiat de référence a été de 360 mg par jour.

Voir tableau 2

Légende du tableau

Cmax observé : Concentration maximale
Tmax observé : Temps au bout duquel on observe le Cmax
T 1/2 : Temps de demi-vie d'élimination
AUC cumulée : Aire sous la courbe figurant la quantité de Diltiazem absorbée en 24 heures
l'AUC cumulée est calculée par la méthode des trapèzes
AUC extrapolée : Aire sous la courbe extrapolée à l'infini

TABLEAU 2

| Produit | Protocole | Cmax observé en ng/ml | Tmax observé en heure | T 1/2 en heure | AUC cumulée en ng/h/ml | AUC extrapolé ng/h/ml |
|---|---|---|---|---|---|---|
| 180 mg SR | Jour 1 après prise unique | 99,4 | 5,7 | 7,45 | 1298,5 | 1537,2 |
| | Jour 7 (équilibre) après prise unique | 125,5 | 4,7 | 7,3 | 1695,0 | 1746,2 |
| 60 mg référence | Jour 1 après trois prises | 61,5 | 16,5 | 31,1 | 977,5 | 2177,8 |
| | Jour 7 (équilibre) après prise unique | 100,0 | 2,3 | 6,5 | 850,7 | 886,0 |
| 240 mg SR | Jour 1 après prise unique | 141,2 | 5,9 | 7,9 | 1811,3 | 2291,2 |
| | Jour 7 (équilibre) après prise unique | 178,1 | 4,95 | 5,5 | 2424,95 | 2529,3 |
| 60 mg référence | Jour 1 après 2 prises de 120 mg | 103,4 | 7,4 | 16,4 | 1387,7 | 1998,5 |
| | Jour 7 (équilibre) après prise unique de 120 mg | 172,2 | 2,7 | 5,7 | 1507,5 | 1582,8 |
| 300 mg SR | Jour 1 après prise unique | 142,4 | 7,1 | 10,0 | 1877,2 | 2567,7 |
| | Jour 7 (équilibre) après prise unique | 201,6 | 6,5 | 7,9 | 3090,2 | 3176,1 |
| 50 mg référence | Jour 1 après prise unique de 120 mg | 77,7 | 4,2 | 5,3 | 687,3 | 754,6 |
| | Jour 7 (équilibre) après prise unique de 120 mg | 176,8 | 3,1 | 6,6 | 1843,7 | 1913,1 |

Par ailleurs, la figure 3 montre la concentration plasmatique du Diltiazem [C] exprimée en nanogrammes par millilitre en fonction du temps [T] en heures le premier jour (courbe n° 1 pour le TILDIEM® et courbe n° 2 pour le produit selon l'invention), et la figure 4 la même courbe le septième jour d'administration pour un dosage à 300 mg selon l'invention en comparaison avec trois fois 120 mg en trois prises de TILDIEM® pendant six jours et une fois 120 mg le septième jour. Les figures 5 et 6 montrent la

comparaison d'un dosage à 180 mg selon l'invention en comparaison avec le TILDIEM® 60 mg (administré trois fois du jour 1 au jour 6, puis une fois au jour 7), respectivement le premier jour et le septième jour, tandis que les figures 7 et 8 comparent un dosage à 240 mg selon l'invention en comparaison à 240 mg de TILDIEM® en deux prises pendant six jours, puis 120 mg le septième jour.

L'ensemble de ces résultats montre bien qu'avec les formes galéniques selon l'invention, on obtient toujours un taux circulant de Diltiazem dans le sang supérieur à 40 nanogrammes par millilitre et, par conséquent, on peut utiliser ces formes en une seule prise par jour.

Dans une seconde série d'exemples, on a utilisé comme constituant principal de la membrane externe un mélange d'Aquacoat® ECD 30 et de Dibutylsébaçate. L'Aquacoat® est une dispersion polymérique aqueuse d'éthylcellulose, commercialisée par la société américaine FMC, qui contient 30 % de composants solides L'éthylcellulose représente 85 % des composants solides, le reste étant constitué de deux stabilisants : le lauryl sulfate de sodium et l'alcool cétylique.

Dans les formulations ci-après, la plastifiant (par exemple le dibutylsébaçate) est utilisé à raison d'une quantité égale à 15 à 25 % de la masse d'Aquacoat exprimée en poids sec. Les caractéristiques de la membrane externe des microgranules ainsi obtenues doivent permettre de libérer le Diltiazem en milieu aqueux selon la méthode américaine USP n° 21 selon les intervalles suivants:

15 à 35 % au bout d'une heure
55 à 75 % au bout de quatre heures
75 à 95 % au bout de huit heures.

En utilisant une suspension aqueuse à 30 % d'éthylcellulose selon des proportions exprimées en poids sec de 100 parties en poids et de 25 parties en poids de dibutylsébaçate, les limites souhaitables de chaque composant utilisé figurent dans le tableau 3, ci-après exprimés en proportion de poids sec par rapport au poids total de produit fini (microgranules vrac).

TABLEAU 3

| COMPOSANTS | PROPORTION % |
|---|---|
| Granules neutres | 8-15 |
| Diltiazem | 60-85 |
| Polyvinylpyrrolidone | 4-6 |
| Aquacoat® ECD 30 | 1-15 |
| Dibutylsébaçate | 0,4-4 |
| Talc | 0-10 |

De la même façon qu'avec la couche externe à base de gomme laque, on a indiqué sur la figure 2 les courbes de délitement in vitro (méthode américaine USP n° 21) les limites correspondant aux intervalles cités plus haut (courbes n° 1 et n° 2), ainsi que trois exemples de formulation sans talc, le premier (courbe n° 3) comportant 8 % en poids sec de mélange Aquacoat® ECD 30 et Dibutylsébaçate (DBS), le second (courbe n° 4) comportant 8,8 % de ECD 30 et DBS, et le troisième (courbe n° 5) comportant 9,8 % de ECD 30 et DBS. Chacune de ces courbes étant un peu plus lente à chaque ajout d'excipient. Une formulation comportant 15 % d'Aquacoat® et de DBS est trop lente (courbe n° 6)

Afin de bien montrer la bio-équivalence des formulations comportant le mélange Aquacoat® et DBS par rapport à celles comportant le mélange gomme laque et éthylcellulose dans leurs membranes externes, on a fait apparaître sur la figure 9 les courbes exprimant la concentration plasmatique du Diltiazem [C] en nanogrammes par millilitre en fonction du temps [T] en heures lors de l'administration de 300 mg de Diltiazem en microgranules selon l'invention en prise unique sur vingt-et-un volontaires, la courbe 1 se rapportant à une formule comportant de la gomme laque et de l'ethylcellulose conforme à la courbe 4 de la figure 1, la courbe 2 se rapportant à la formulation conforme à la courbe 5 de la figure 2 et la courbe 3 se rapportant à la formulation conforme à la courbe 3 de la figure 2.

Sur la figure 10, sont reproduites les courbes moyennes d'une étude ayant démontré l'inéquivalence entre une formule comportant de la gomme laque (courbe n° 1) conforme à la courbe 4 de la figure 1 et une formule à l'Aquacoat® (courbe n° 2) conforme à la courbe 6 de la figure 2.

Enfin, on a fait apparaître à la figure 11 les résultats obtenus lors d'une étude en prise unique sur dix-huit volontaires comparant 180 mg de la formulation du tableau 3 (courbe n° 1) en comparaison avec trois fois 60 mg de TILDIEM® (courbe n° 2)

L'ensemble des résultats de ces formulations est résumé dans le tableau IV ci-après.

TABLEAU 4

| | Cmax observé en ng/ml | Tmax observé en heure | T 1/2 en heure | AUC cumulée en ng/h/ml | AUC extrapolée ng/h/ml |
|---|---|---|---|---|---|
| 300 mg SR formulation gomme laque | 218,5 | 4,2 | 5,5 | 2636,1 | 3154,3 |
| 300 mg SR formulation aquacoat (courbe 3 de la figure 9) | 275,5 | 3,0 | 7,2 | 2792,5 | 3161,1 |
| 300 mg SR formulation aquacoat (Courbe 2 de la figure 9) | 201,5 | 5,6 | 7,4 | 2484,5 | 2878,7 |
| 180 mg SR formule aquacoat | 102,15 | 5,5 | 6,4 | 1160,1 | 1193,3 |
| 180 mg référence | 176,95 | 3,75 | 5,7 | 1381,9 | 1397,7 |

Légende

Cmax observé : Concentration maximale
Tmax observé : Temps au bout duquel on observe le Cmax
T 1/2 : Temps de demi-vie d'élimination
AUC cumulée : Aire sous la courbe figurant la quantité de Diltiazem absorbée en 24 heures

l'AUC cumulée est calculée par la méthode des trapèzes
AUC extrapolée : Aire sous la courbe extrapolée à l'infini

La membrane externe des produits se rapportant au tableau 4 presente l'avantage d'obtenir des formulations dont la liberation du Diltiazem est insensible au pH.

Bien entendu, des résultats équivalents peuvent être obtenus en utilisant d'autres excipients tels que la suspension d'ETHOCEL AQ commercialisée par la société COLORCON, les EUDRAGIT® RL et RS ou leurs equivalents commercialises par le societe ROHM et HAAS, les formules équivalentes selon l'invention

à celles décrites ci-dessus étant aisément réalisées par l'homme de l'art, ces formules devant permettre d'obtenir des résultats bio-équivalents à l'une ou l'autre des formulations ci-dessus.

Toutes les formes galéniques selon l'invention permettent de conserver une concentration maximale sanguine efficace suffisante sans pic de concentration excessif de façon à permettre une prise par jour quelle que soit l'indication traitee (soit entre 40 et 300 nanogrammes de Diltiazem par millilitre de sang prélevé), c'est-à-dire en faisant varier la dose journalière unique à faire absorber au patient. Elles permettent donc d'obtenir un avantage décisif dans l'administration du Diltiazem ou de ses sels pharmaceutiquement acceptables. Les dosages habituels pourront varier sans inconvénient entre 50 et 500 mg par jour, en une ou deux prises. Pour deux prises, on préfèrera une dose de 50 à 200 mg et pour une prise de 120 à 400 mg suivant que l'on traite l'angine de poitrine, l'hypertension ou l'insuffisance circulatoire cérébrale ou périphérique.

La toxicité des formulations comparée à celle du chlorhydrate de Diltiazem sous forme de poudre n'a pas montre d'accroissement quelconque et au contraire une diminution par rapport au produit pur Diltiazem.

Par ailleurs, la stabilité des formulations s'est révélée excellente, notamment pour les formulations du second groupe comportant l'Aquacoat®. Pour celles comportant de la gomme laque, on a constaté qu'il était essentiel d'éliminer toutes traces de solvant à l'issue de la fabrication. Dans tous les cas, on a intérêt à effectuer un chauffage à environ 50 ° C de la masse finale des microgranules avant leur mise en gélule, que ceux-ci aient été préparés selon le procédé classique en turbine ou par extrusion plus sphéronisation et enrobés en turbine ou à l'aide de variantes à lits d'air fluidisés par exemple du type GLATT, WURSTER, bien connus de l'homme de l'art, et qui peuvent permettre d'utiliser directement le principe actif granulé comme élément du centre des microgranules.

**Revendications**

1. Forme galénique à libération prolongée de Diltiazem pharmaceutiquement acceptable comportant des microgranules, du type constitué en son centre d'un noyau garni de couches comportant le principe actif, chaque microgranule comportant une membrane externe, caractérisée en ce que celle-ci est adaptée pour libérer le Diltiazem appliqué sur le noyau neutre dans un milieu aqueux selon des proportions suivantes, mesurées selon la méthode de la pharmacopée américaine USP n ° 21
   a) entre 5 et 35 % après une heure
   b) entre 15 et 40 % après deux heures
   c) entre 20 et 50 % après trois heures
   d) entre 30 et 75 % après quatre heures
   e) entre 40 et 80 % après six heures
   d) entre 55 et 95 % après huit heures.

   et en ce qu'elle comporte, exprimé en poids total de micro-granules :

| | |
|---|---|
| - noyau neutre → | 8 à 15 % |
| - couche active constituée de | |
| Diltiazem →<br>Polyvinylpyrrolidone →<br>- membrane externe → | 60 à 85 %<br>4 à 6 %<br>1 à 20 % |

2. Forme galénique selon la revendication 1, caractérisée en ce que la membrane extérieure comporte, exprimée en poids total de microgranules, de 1 à 15% en poids d'éthylcellulose et de 0,1 à 4 % en poids de plastifiant.

3. Forme galénique selon la revendication 1, caractérisée en ce que la membrane extérieure comporte, exprimée en poids total de microgranules, de 2 à 5% de gomme laque et de 1 à 3 % d'éthylcellulose.

4. Forme galénique selon la revendication 1, caractérisée en ce que la membrane exterieure de chaque microgranule comporte une proportion de talc inférieure à 10 % en poids total de microgranules.

5. Forme galénique selon une des revendications 1 à 4, caractérisée en ce qu'elle est adaptée à libérer le Diltiazem appliqué sur le noyau neutre dans un milieu aqueux selon des proportions suivantes,

mesurées selon la méthode de la pharmacopée américaine USP n° 21.

a) entre 5 et 35 % après une heure
b) entre 15 et 40 % après deux heures
c) entre 20 et 50 % après trois heures
d) entre 30 et 75 % après quatre heures
e) entre 40 et 80 % après six heures
f) entre 55 et 95 % après huit heures.

**6.** Forme galénique selon la revendication 1, caractérisée en ce que la membrane externe est constituée à l'aide d'une solution organique du type alcool éthylique ou acétone, de gomme laque et d'éthylcellulose selon des proportions respectives de 70 parties en poids de gomme laque et 30 parties en poids d'éthylcellulose exprimées en poids sec.

**7.** Forme galénique selon la revendication 6, caractérisée en ce que les proportions de Diltiazem libérées en milieu aqueux mesurées selon la méthode américaine USP n° 21 sont sensiblement comprises entre :

10 à 20 % au bout d'une heure
30 à 45 % au bout de quatre heures
60 à 75 % au bout de huit heures.

**8.** Forme galénique selon la revendication 1, caractérisée en ce que la membrane externe est constituée d'un plastifiant du type dibutylsébaçate introduit dans une suspension aqueuse à 30 % d'éthylcellulose selon des proportions respectives de 100 parties en poids d'éthylcellulose et de 15 à 25 parties en poids de plastifiant exprimées en poids sec.

**9.** Forme galénique selon la revendication 8, caractérisée en ce que les proportions de Diltiazem libérées en milieu aqueux mesurées selon la méthode américaine USP n° 21 sont sensiblement comprises entre :

15 à 35 % au bout d'une heure
55 à 75 % au bout de quatre heures
75 à 95 % au bout de huit heures.

**10.** Procédé d'obtention de la forme galénique conforme à la revendication 1 selon lequel on utilise un noyau neutre classique dont la taille est comprise entre 0,2 et 2 mm, on applique des couches de Diltiazem et/ou de ses sels pharmaceutiquement acceptables, caractérisé en ce que cette application est réalisée à l'aide d'une solution de polyvinylpyrrolidone dans l'eau ou un solvant avec laquelle on mouille préalablement des microgranules, puis on projette le principe actif et on mélange avec ladite solution jusqu'à épuisement total de la quantité correspondant au titre recherché, lesdits microgranules étant séchés jusqu'à évaporation totale du solvant.

**11.** Procédé selon la revendication 10, caractérisé en ce que, pour former une couche externe d'enrobage, on prépare une solution organique de gomme laque et d'éthylcellulose et une quantité appropriée de talc, puis on applique la solution organique de gomme laque et d'éthylcellulose et on projette simultanément le talc et on sèche à l'air chaud jusqu'à évaporation du solvant.

**12.** Procédé selon la revendication 10, caractérisé en ce que, pour former la couche extérieure d'enrobage, on prépare une suspension d'Aquacoat® et de plastifiant et une quantité appropriée de talc, puis on applique la suspension d'Aquacoat® et de plastifiant et on projette simultanément le talc et on sèche à l'air chaud jusqu'à évaporation totale des solvants.

**13.** Médicament selon la revendication 1, caractérisé en ce qu'il comporte les microgranules inclus dans une présentation unitaire du type gélule -sachet ou comprimé- dosée entre 50 et 250 mg de Diltiazem et destinée à être absorbée deux fois par jour pour le traitement de l'angor, de l'hypertension ou de l'insuffisance circulatoire cérébrale et périphérique.

**14.** Médicament selon la revendication 1, caractérisé en ce qu'il comporte les microgranules inclus dans une présentation unitaire du type gélule -sachet ou comprimé- dosée entre 100 et 500 mg de Diltiazem et destinée à être absorbée une fois par jour pour le traitement de l'angor, de l'hypertension ou de

l'insuffisance circulatoire cérébrale et périphérique.

**15.** Médicament selon la revendication 14, caractérisé en ce qu'il comporte les microgranules inclus dans une présentation unitaire du type gélule -sachet ou comprimé- dosée à 300 mg de Diltiazem et destinée à être absorbée une fois par jour pour le traitement de l'hypertension.

**16.** Médicament selon l'une des revendications 13 ou 14, caractérisé en ce que les taux sanguins circulants obtenus à l'équilibre pendant une journée sont compris entre sensiblement 40 et 300 nanogrammes par millilitre de sang prélevé.

## Claims

**1.** Slow release Galenical preparation of pharmaceutically acceptable Diltiazem comprising microgranules of the type constituted by a central core coated with layers containing the active substance, with each microgranule having an outer membrane, characterized in that the preparation is adapted to release the Diltiazem applied to the neutral core into an aqueous medium at the following rates measured using the method of the United States Pharmacopeia No. 21:
(a) between 5% and 35% after one hour;
(b) between 15% and 40% after two hours;
(c) between 20% and 50% after three hours;
(d) between 30% and 75% after four hours;
(e) between 40% and 80% after six hours;
(f) between 55% and 95% after eight hours;
whereas said preparation comprises, relative to the total weight of microgranules, 8% to 15% by weight of neutral core, active layers of Diltiazem comprising 60% to 85% by weight mixed with polyvinylpyrrolidone comprising 4% to 6% by weight, and 1% to 20% by weight outer membrane.

**2.** Galenical preparation according to claim 1, characterized in that the outer membrane includes, expressed in terms of total microgranule weight, 1% to 15% ethylcellulose and 0,1% to 4% of plasticizer.

**3.** Galenical preparation according to claim 1, characterized in that the outer membrane includes, expressed in terms of total microgranule weight, 2% to 5% shellac and 1% to 3% ethylcellulose.

**4.** Galenical preparation according to claim 1, characterized in that the outer membrane of each microgranule includes not more than 10% talc, the percentage being relative to the total weight of microgranules.

**5.** Galenical preparation according to one of claims 1 to 4, characterized in that it is adapted to release the Diltiazem applied to the neutral core into an aqueous medium at the following rates measured using the method of the United States Pharmacopeia No. 21:
(a) between 5% and 35% after one hour;
(b) between 15% and 40% after two hours;
(c) between 20% and 50% after three hours;
(d) between 30% and 75% after four hours;
(e) between 40% and 80% after six hours;
(f) between 55% and 95% after eight hours.

**6.** Galenical preparation according to claim 1, characterized in that the outer membrane is constituted by means of an organic solution of acetone or ethyl alcohol type containing shellac and ethylcellulose with 70 parts shellac to 30 parts ethylcellulose, said parts being parts by dry weight.

**7.** Galenical preparation according to claim 6, characterized in that the Diltiazem is released into an aqueous medium as measured using the method of USP No. 21 at rates lying substantially between:
(a) 10% to 20% after one hour;
(b) 30% to 45% after four hours;
(c) 60% to 75% after eight hours.

**8.** Galenical preparation according to claim 1, characterized in that the outer membrane is constituted by a plasticizer of the dibutylsebacate type inserted in an aqueous suspension having 30 percent ethylcellulose with 100 parts by weight ethylcellulose and 15 to 25 parts per weight plasticizer with the weights being expressed in terms of dry weight.

**9.** Galenical preparation according to claim 8, characterized in that the Diltiazem is released into an aqueous medium as messured using the method of USP No. 21 at a rate lying substantially between:
(a) 15% to 35% after one hour;
(b) 55% a 75% after four hours;
(c) 75% to 95% after eight hours.

**10.** Method for obtaining a Galenical preparation according to claim 1, in which a conventional neutral core is used having a size lying in the range 0.2 mm to 2 mm, in which layers of Diltiazem and/or pharmaceutically acceptable salts thereof are applied to the core, characterized in that said application is performed by means of a solution of polyvinylpyrrolidone in water or a solvent with which the microgranules are initially wetted, after what the active substance is projected and mixed with said solution until the entire quantity of active substance corresponding to the desired dose has been used up, with said microgranules being dried until the solvent has been completely evaporated.

**11.** Method according to claim 10, characterized in that in order to form an outer coating layer, an organic solution of shellac and ethylcellulose is prepared together with an appropriate quantity of talc, after what the organic solution of shellac and ethylcellulose is applied while the talc is simultaneously projected, and drying is then performed in hot air until the solvent has evaporated.

**12.** Method according to claim 10, characterized in that in order to form the outer coating layer, a suspension of Aquacoat and plasticizer is prepared together with an appropriate quantity of talc, after what the suspension of Aquacoat and plasticizer is applied and the talc is simultaneously projected, and drying is performed in hot air until the solvents have completely evaporated.

**13.** Medicine according to claim 1, characterized in that the microgranules are contained in a unit preparation of the capsule, sachett, or pill type, at a dose between 50 mg and 250 mg of Diltiazem per unit and intended to be taken twice a day for treating angina pectoris, hypertension, or peripheral or cerebral circulatory insufficiency.

**14.** Medicine according to claim 1, characterized in that the microgranules are contained in a unit preparation of the capsule, sachett or pill type, at a dose between 100 mg and 500 mg of Diltiazem per unit and intended to be taken once a day for treating angina pectoris, hypertension, or peripheral or cerebral circulatory insufficiency.

**15.** Medicine according to claim 14, characterized in that it includes the microgranules placed in a unit preparation of the capsule, sachett, or pill type, at a dose of 300 mg of Diltiazem per unit and intended to be taken once a day for treating hypertension.

**16.** Medicine according to one of claims 13 and 14, characterized in that the equilibrium concentrations obtained in the blood flow over a day lies substantially in the range 40 to 300 nanograms per milliliter of blood sampled.

**Patentansprüche**

**1.** Galenische Zubereitung zur verlängerten Freisetzung von pharmazeutisch aufnehmbarem Diltiazem, die Mikrokörner umfaßt, die in ihrem Zentrum aus einem Kern bestehen, der mit Schichten versehen ist, die den Wirkstoff umfassen, wobei jedes Mikrokorn eine äußere Membran aufweist, dadurch **gekennzeichnet,** daß die Zubereitung so ausgebildet ist, daß sie das auf den neutralen Kern aufgebrachte Diltiazem in einem wässrigen Milieu in den folgenden Verhältnissen freisetzt, die nach dem Verfahren des amerikanischen Arzneibuches USP 21 gemessen sind:
a) zwischen 5 und 35 % nach einer Stunde,
b) zwischen 15 und 40 % nach zwei Stunden,
c) zwischen 20 und 50 % nach drei Stunden,

d) zwischen 30 und 75 % nach vier Stunden,
e) zwischen 40 und 80 % nach sechs Stunden,
f) zwischen 55 und 95 % nach acht Stunden

und daß sie, bezogen auf das Gesamtgewicht der Mikrokörner folgende Anteile enthält:

| | |
|---|---|
| - neutraler Kern | 8 bis 15 % |
| - aktive Schicht aus | |
| Diltiazem<br>Polyvinylpyrrolidon<br>- äußere Membran | 60 bis 85 %<br>4 bis 6 %<br>1 bis 20 %. |

**2.** Galenische Zubereitung nach Anspruch 1, dadurch **gekennzeichnet,** daß die äußere Membran bezogen auf das Gesamtgewicht der Mikrokörner 1 bis 15 Gew.% Äthylzellulose und 0,1 bis 4 Gew.% Weichmacher enthält.

**3.** Galenische Zubereitung nach Anspruch 1, dadurch **gekennzeichnet,** daß die äußere Membran bezogen auf das Gesamtgewicht der Mikrokörner 2 bis 5 % Gummilack und 1 bis 3 % Äthylzellulose enthält.

**4.** Galenische Zubereitung nach Anspruch 1, dadurch **gekennzeichnet,** daß die äußere Membran eines jeden Mikrokorns einen Anteil an Talk von weniger als 10 % des Gesamtgewichts der Mikrokörner enthält.

**5.** Galenische Zubereitung nach einem der Ansprüche 1 bis 4, dadurch **gekennzeichnet,** daß sie so ausgebildet ist, daß sie das auf den neutralen Kern aufgebrachte Diltiazem in einem wässrigen Milieu in den folgenden Verhältnissen freisetzt, die nach dem Verfahren des amerikanischen Arzneibuches USP 21 gemessen sind:

a) zwischen 5 und 35 % nach einer Stunde,
b) zwischen 15 und 40 % nach zwei Stunden,
c) zwischen 20 und 50 % nach drei Stunden,
d) zwischen 30 und 75 % nach vier Stunden,
e) zwischen 40 und 80 % nach sechs Stunden,
f) zwischen 55 und 95 % nach acht Stunden.

**6.** Galenische Zubereitung nach Anspruch 1, dadurch **gekennzeichnet,** daß die äußere Membran mit Hilfe einer organischen Lösung von Gummilack und Äthylzellulose in Äthylalkohol oder Azeton in folgendem Verhältnis gebildet wird: 70 Gewichtsanteile von Gummilack und 30 Gewichtsanteile Äthylzellulose bezogen auf das Trockengewicht.

**7.** Galenische Zubereitung nach Anspruch 6, dadurch **gekennzeichnet,** daß die Anteile des im wässrigen Milieu freigesetzten Diltiazem gemessen nach dem Verfahren des amerikanischen USP 21 im wesentlichen in folgenden Bereichen liegen:

10 bis 20 % am Ende einer Stunde,
30 bis 45 % am Ende von vier Stunden,
60 bis 75 % am Ende von acht Stunden.

**8.** Galenische Zubereitung nach Anspruch 1, dadurch **gekennzeichnet,** daß die äußere Membran von einem Weichmacher in Art des Dibutylsebazinsäureester gebildet wird, der in eine wässrige Lösung mit 30 % Äthylzellulose in einem solchen Verhältnis eingeführt wird, daß auf 100 Gewichtsteile Äthylzellulose 15 bis 25 Gewichtsteile Weichmacher bezogen auf das Trockengewicht treffen.

**9.** Galenische Zubereitung nach Anspruch 8, dadurch **gekennzeichnet,** daß die Anteile des im wässrigen Milieu freigesetzten Diltiazems gemessen nach dem Verfahren des amerikanischen USP 21 im wesentlichen in folgenden Bereichen liegen:

15 bis 35 % am Ende einer Stunde,

55 bis 75 % am Ende von vier Stunden,
75 bis 95 % am Ende von acht Stunden.

**10.** Verfahren zur Herstellung einer galenischen Zubereitung nach Anspruch 1 bei dem man einen herkömmlichen neutralen Kern verwendet, dessen Größe zwischen 0,2 und 2 mm liegt, wobei man Schichten von Diltiazem und/oder seiner pharmazeutisch aufnehmbaren Salze aufbringt, dadurch **gekennzeichnet,** daß man dieses Aufbringen mit Hilfe einer Lösung von Polyvinlypyrrolidon in Wasser oder einem Lösungsmittel durchführt, mit der man zunächst die Mikrokörner benetzt, worauf man den Wirkstoff herausschleudert und mit der Lösung so lange vermischt, bis die Menge, die dem ermittelten Gehalt entspricht, völlig verbraucht ist, worauf die Mikrokörner so lange getrocknet werden, bis das Lösungsmittel vollständig verdunstet ist.

**11.** Verfahren nach Anspruch 10, dadurch **gekennzeichnet,** daß man zur Herstellung einer äußeren Umhüllungsschicht eine organische Lösung von Gummilack und Äthylzellulose und einer geeigneten Menge von Talk vorbereitet, hierauf die organische Lösung aus Gummilack und Äthylzellulose aufbringt und gleichzeitig den Talk herausschleudert, und daß man mit heißer Luft trocknet, bis das Lösemittel verdunstet ist.

**12.** Verfahren nach Anspruch 10, dadurch **gekennzeichnet,** daß man zur Ausbildung einer äußeren Umhüllungsschicht eine Suspension von Aquacoat® und Weichmacher und eine geeignete Menge von Talk vorbereitet, hierauf die Suspension von Aquacoat® und dem Weichmacher aufbringt und gleichzeitig den Talk herausschleudert, und daß man mit heißer Luft so lange trocknet, bis die Lösemittel vollständig verdunstet sind.

**13.** Medikament nach Anspruch 1, dadurch **gekennzeichnet,** daß es Mikrokörner umfaßt, die in einer einheitlichen Darbietungsform in Art einer Kapsel - in einem Beutel oder als Tablette - enthalten sind und eine Dosis zwischen 50 und 250 mg von Diltiazem aufweisen, und die dazu bestimmt sind, zweimal am Tag eingenommen zu werden, um Angina Pectoris, erhöhten Blutdruck oder eine zerebrale und periphere Blutkreislauf-Insuffizienz zu behandeln.

**14.** Medikament nach Anspruch 1, dadurch **gekennzeichnet,** daß es Mikrokörner umfaßt, die in einer einheitlichen Darbietungsform in Art einer Kapsel - in einem Beutel oder als Tablette - enthalten sind und eine Dosis zwischen 100 und 500 mg von Diltiazem aufweisen, und die dazu bestimmt sind, einmal am Tag eingenommen zu werden, um Angina Pectoris, erhöhten Blutdruck oder eine zerebrale und periphere Blutkreislauf-Insuffizienz zu behandeln.

**15.** Medikament nach Anspruch 14, dadurch **gekennzeichnet,** daß es Mikrokörner umfaßt, die in einer einheitlichen Darbietungsform in Art einer Kapsel - in einem Beutel oder als Tablette - enthalten sind und eine Dosis von 300 mg von Diltiazem aufweisen, und die dazu bestimmt sind, einmal am Tag eingenommen zu werden, um Bluthochdruck zu behandeln.

**16.** Medikament nach Anspruch 13 oder 14, dadurch **gekennzeichnet,** daß die während eines Tages im Gleichgewicht erhaltenen Blutkreislauf-Pegel im wesentlichen zwischen 40 und 300 Nanogramm pro Milliliter entnommenen Blutes liegen.

Fig: 1

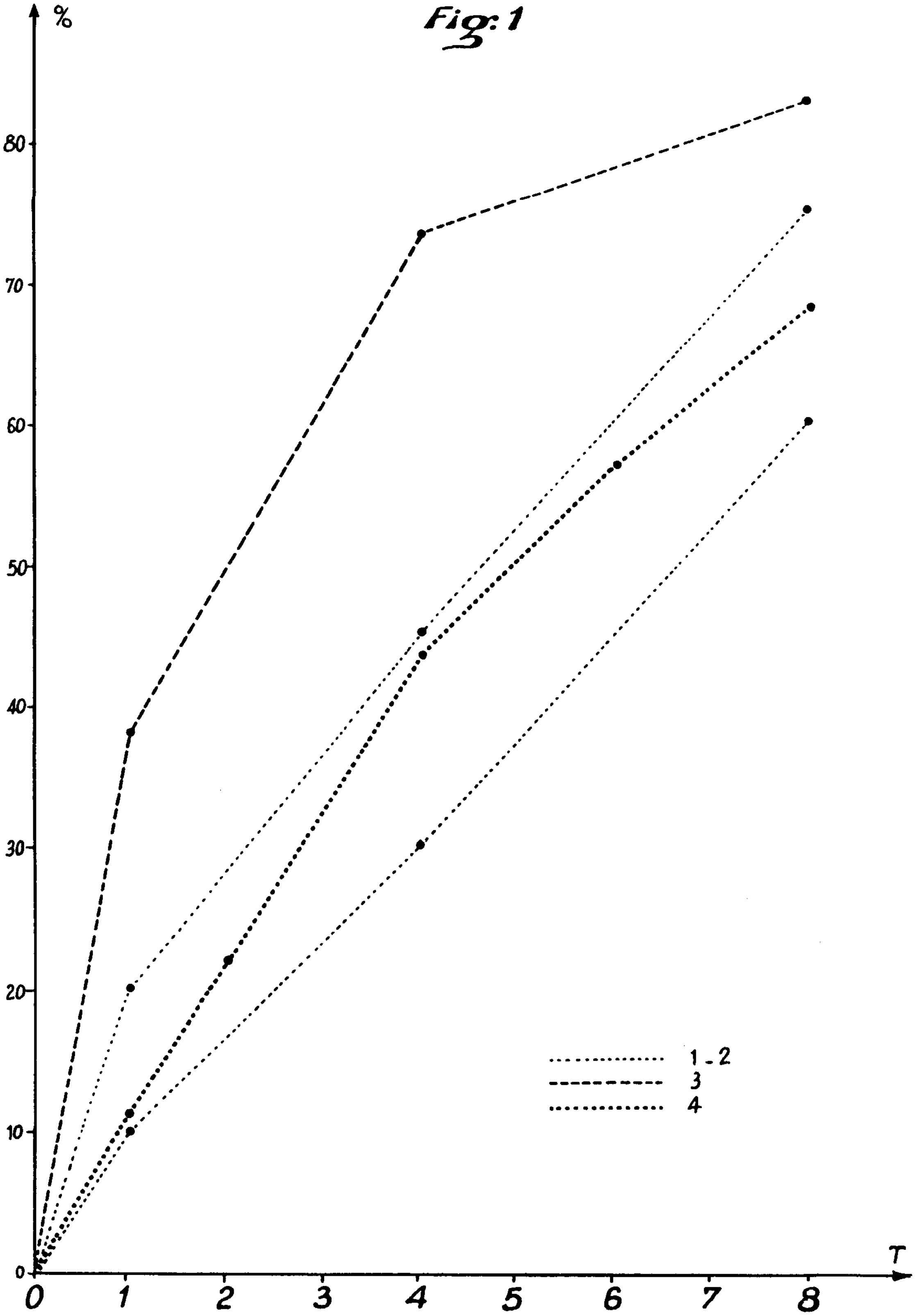

Fig: 2

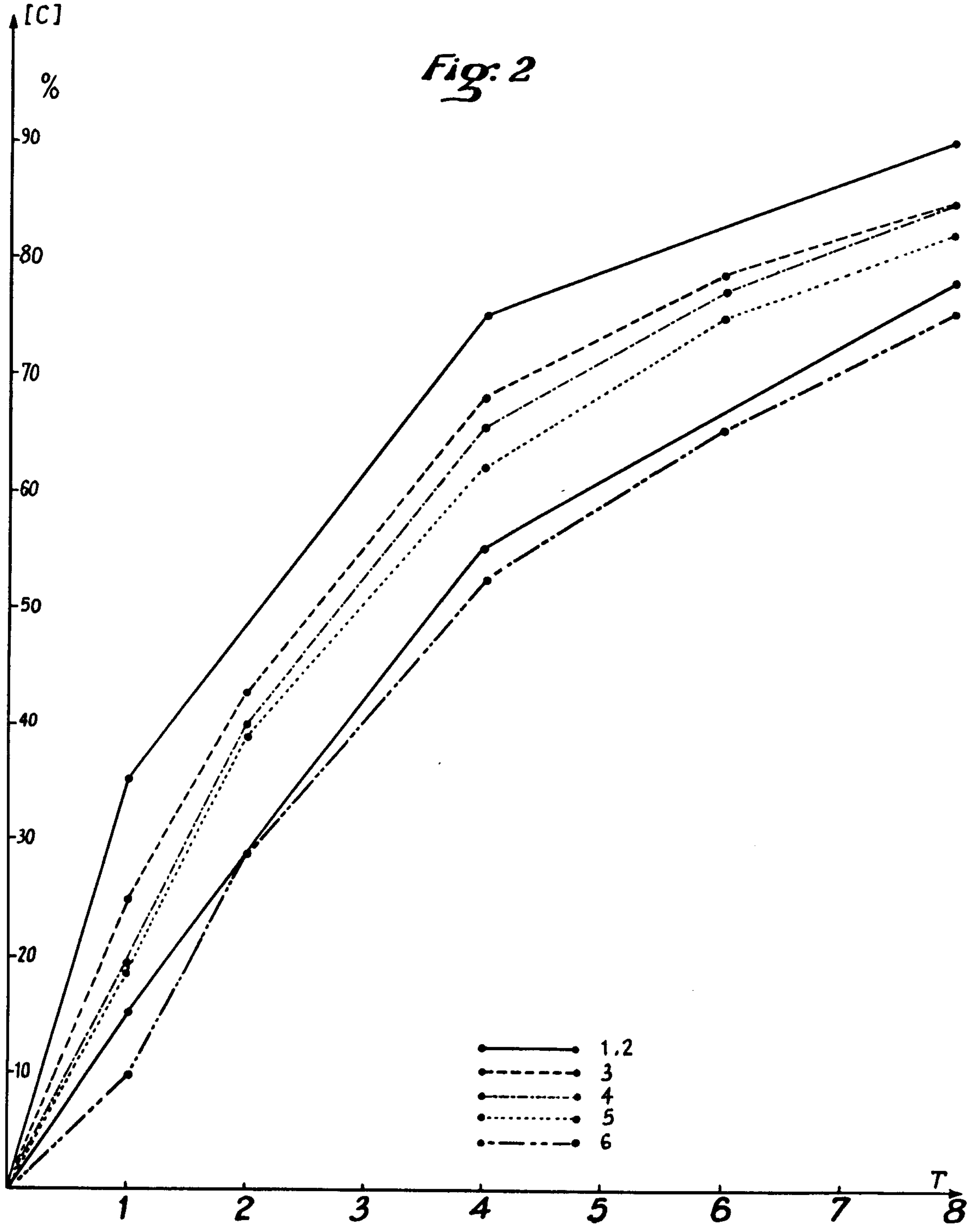

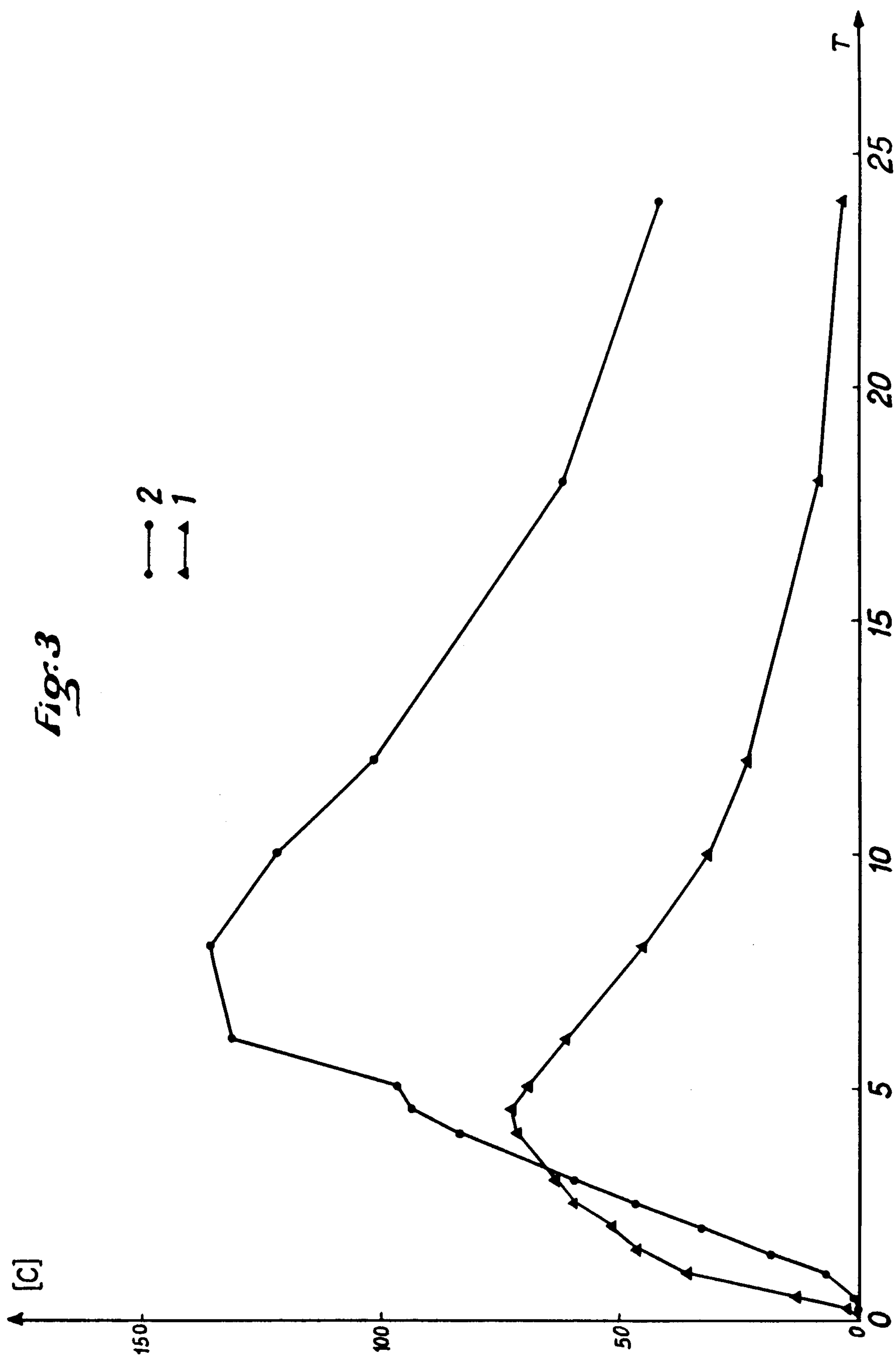


Fig. 3

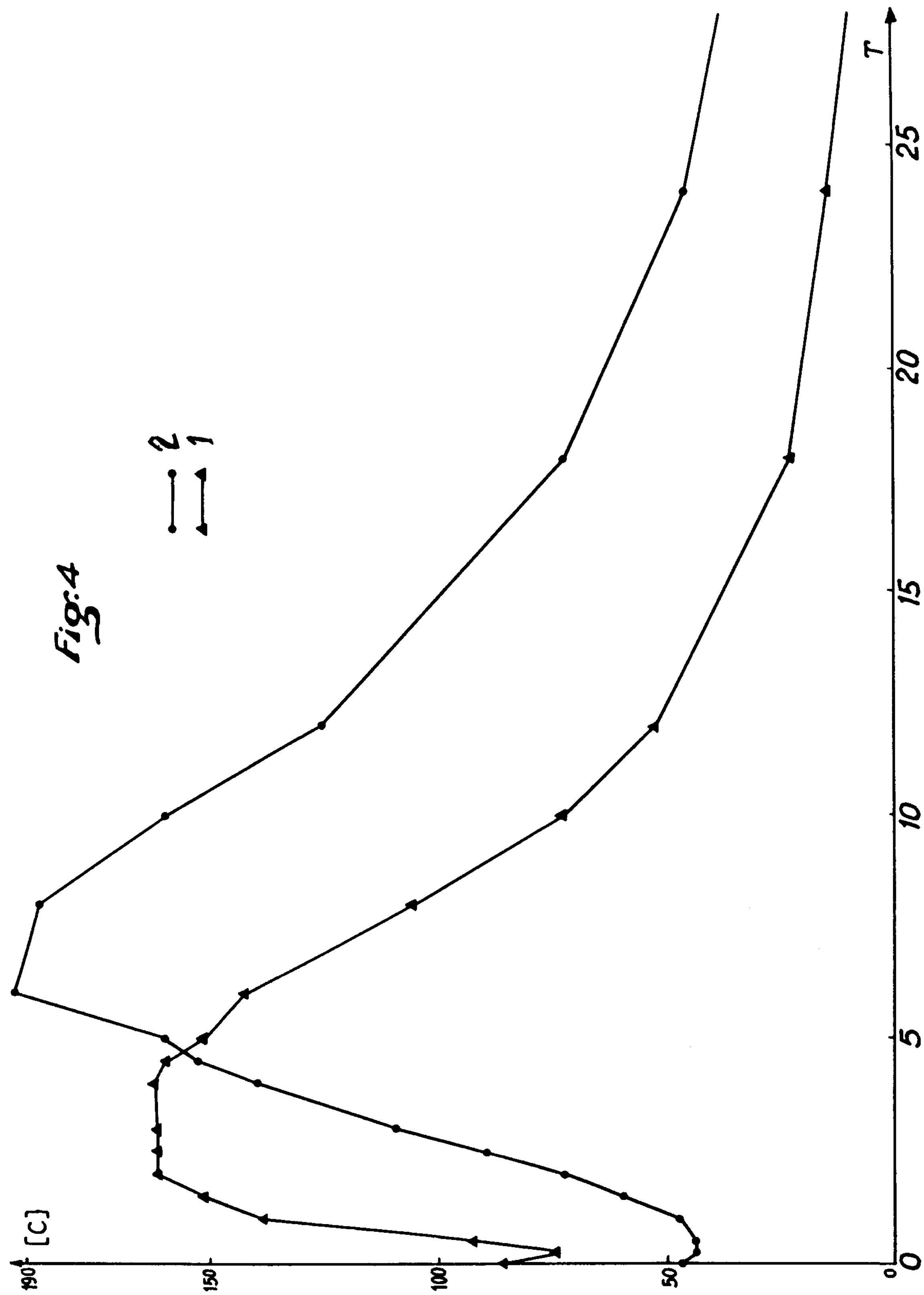


Fig:4

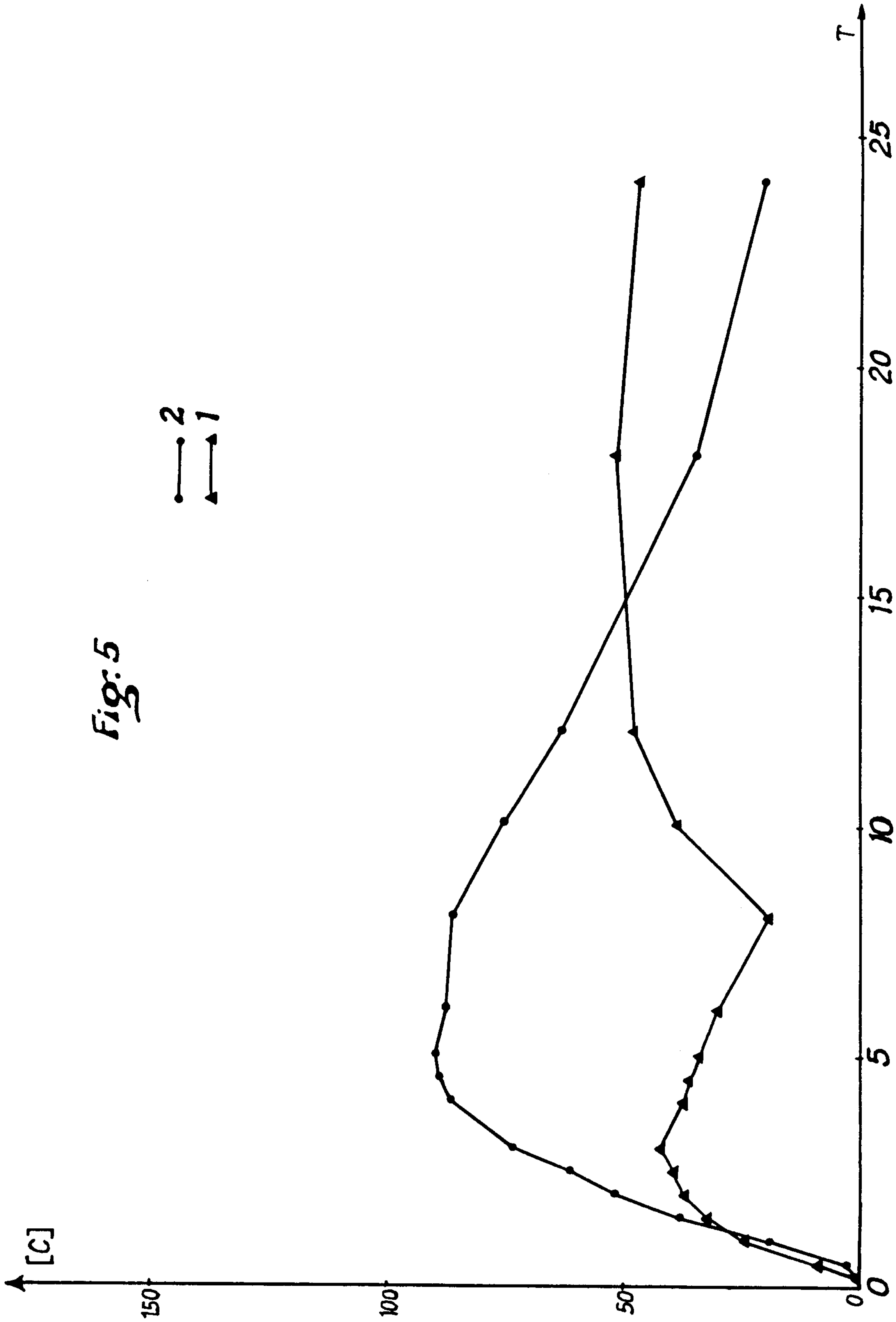


Fig: 5

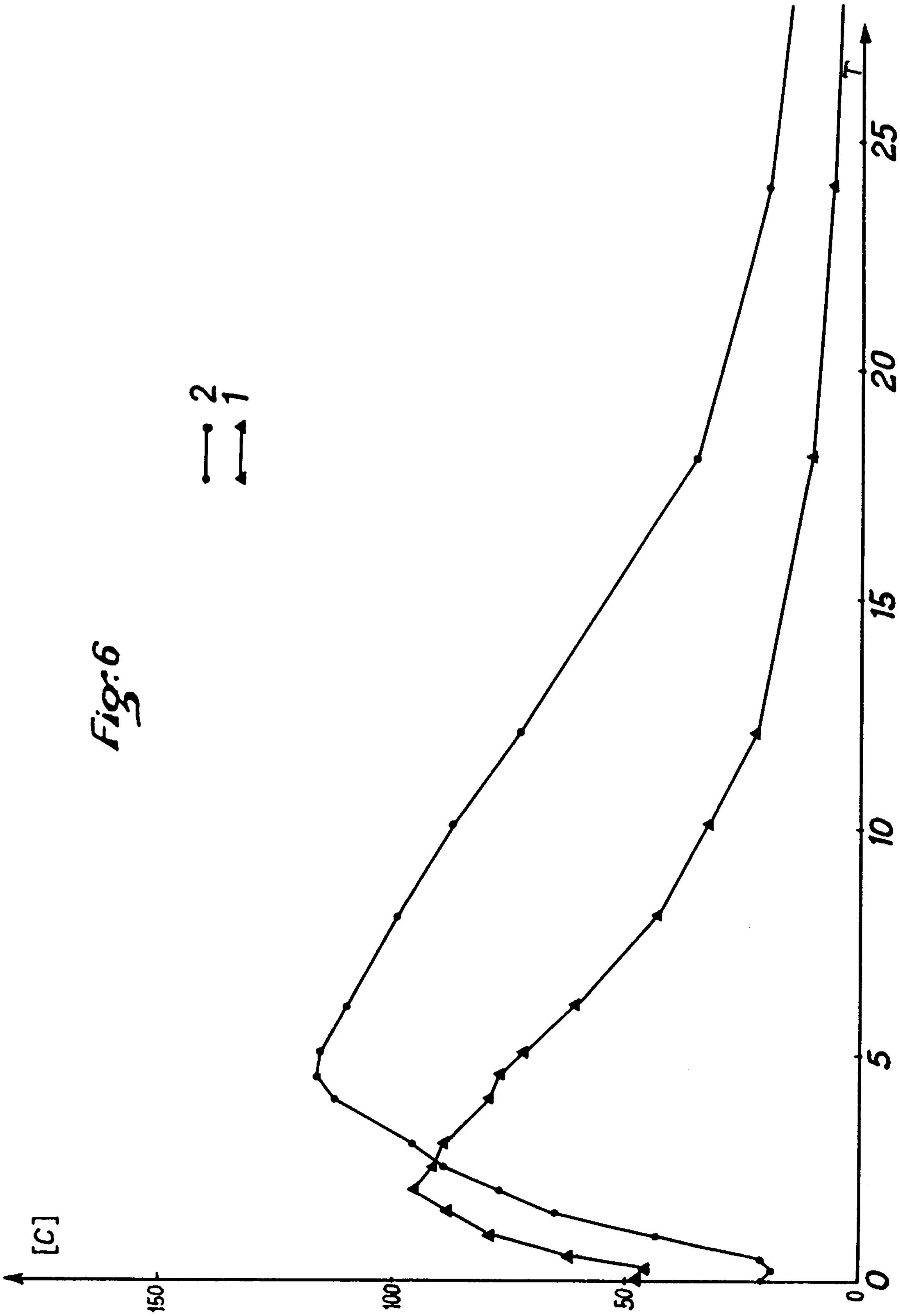


Fig:6

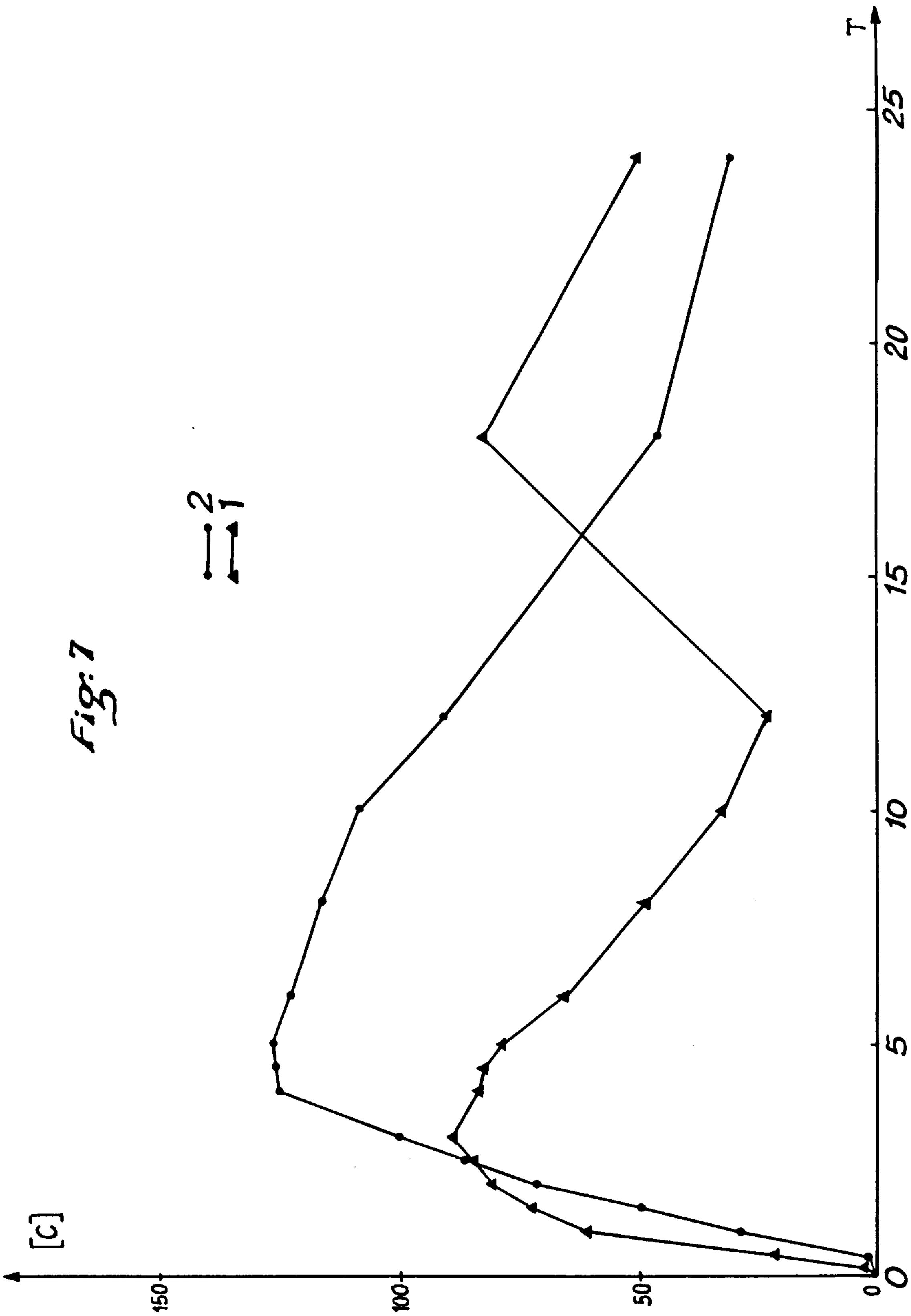


Fig. 7

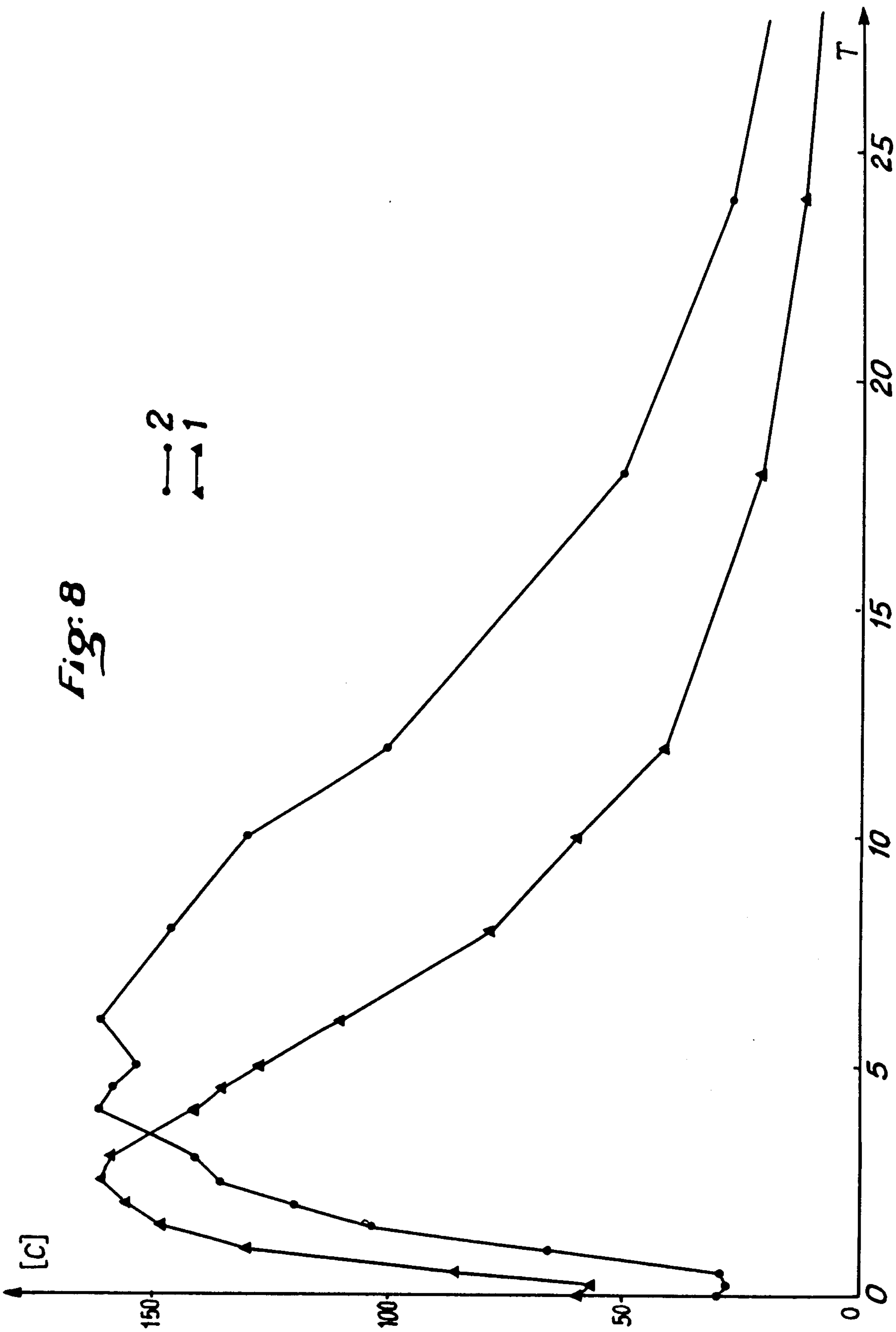


Fig. 8

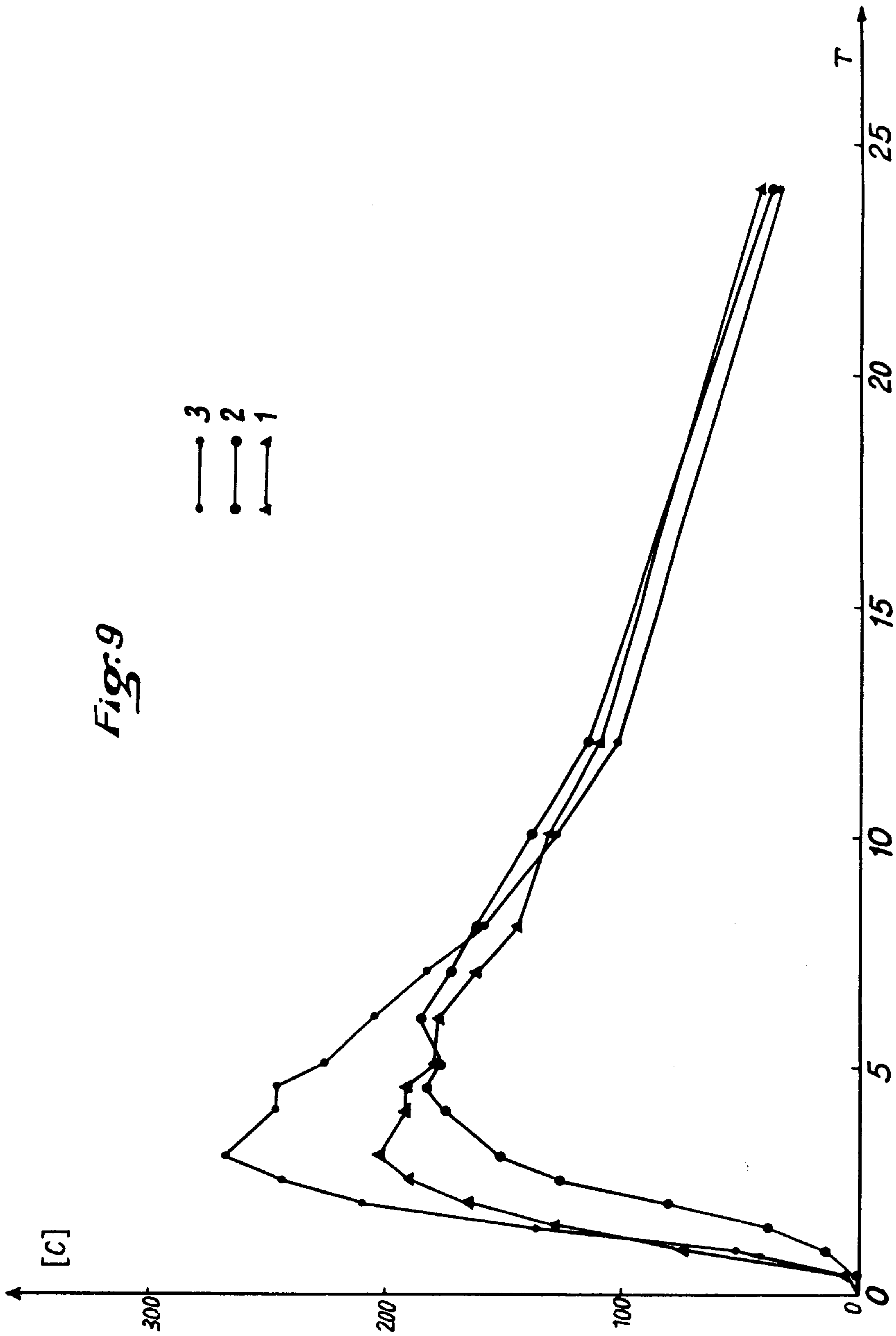


Fig. 9

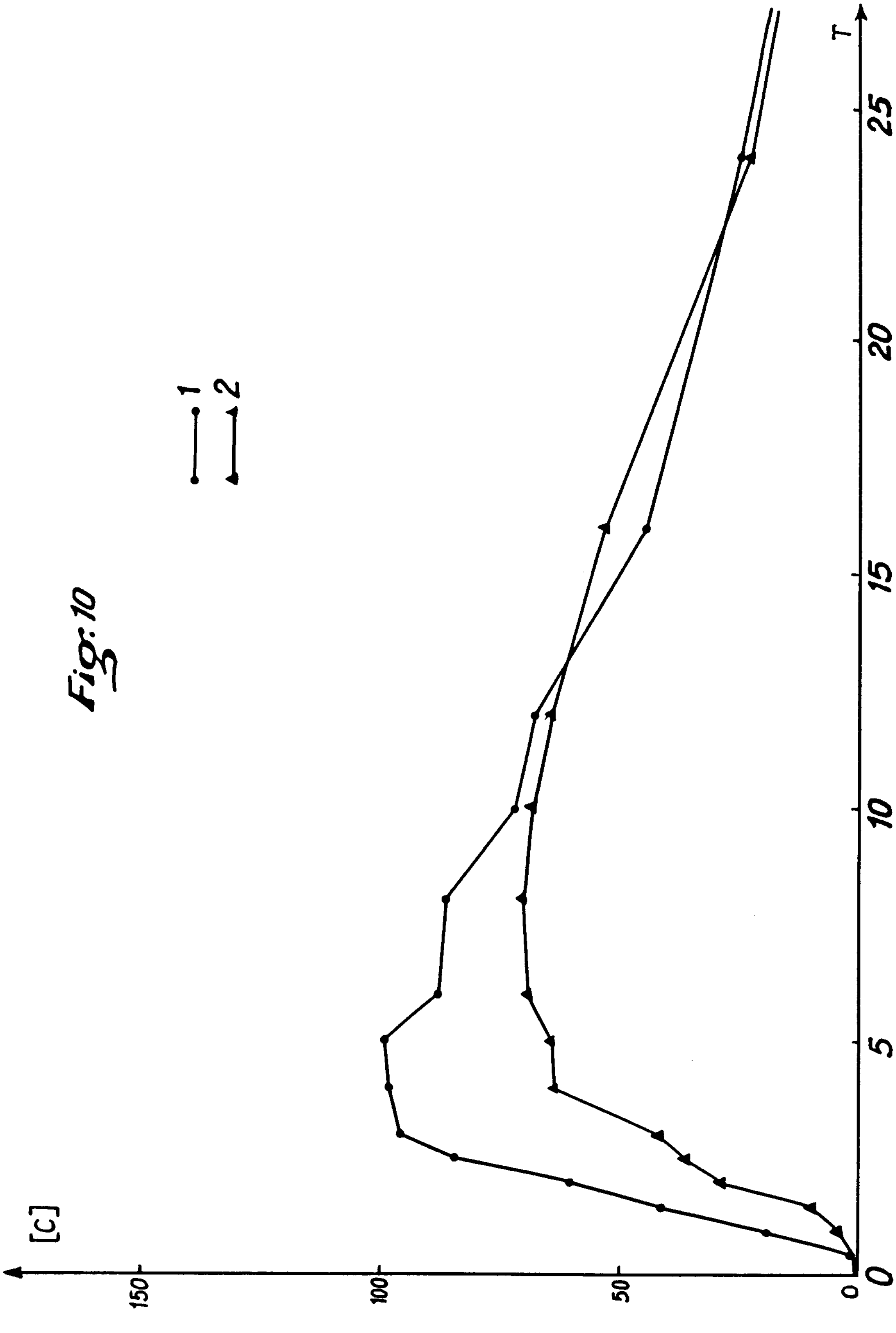


Fig. 10

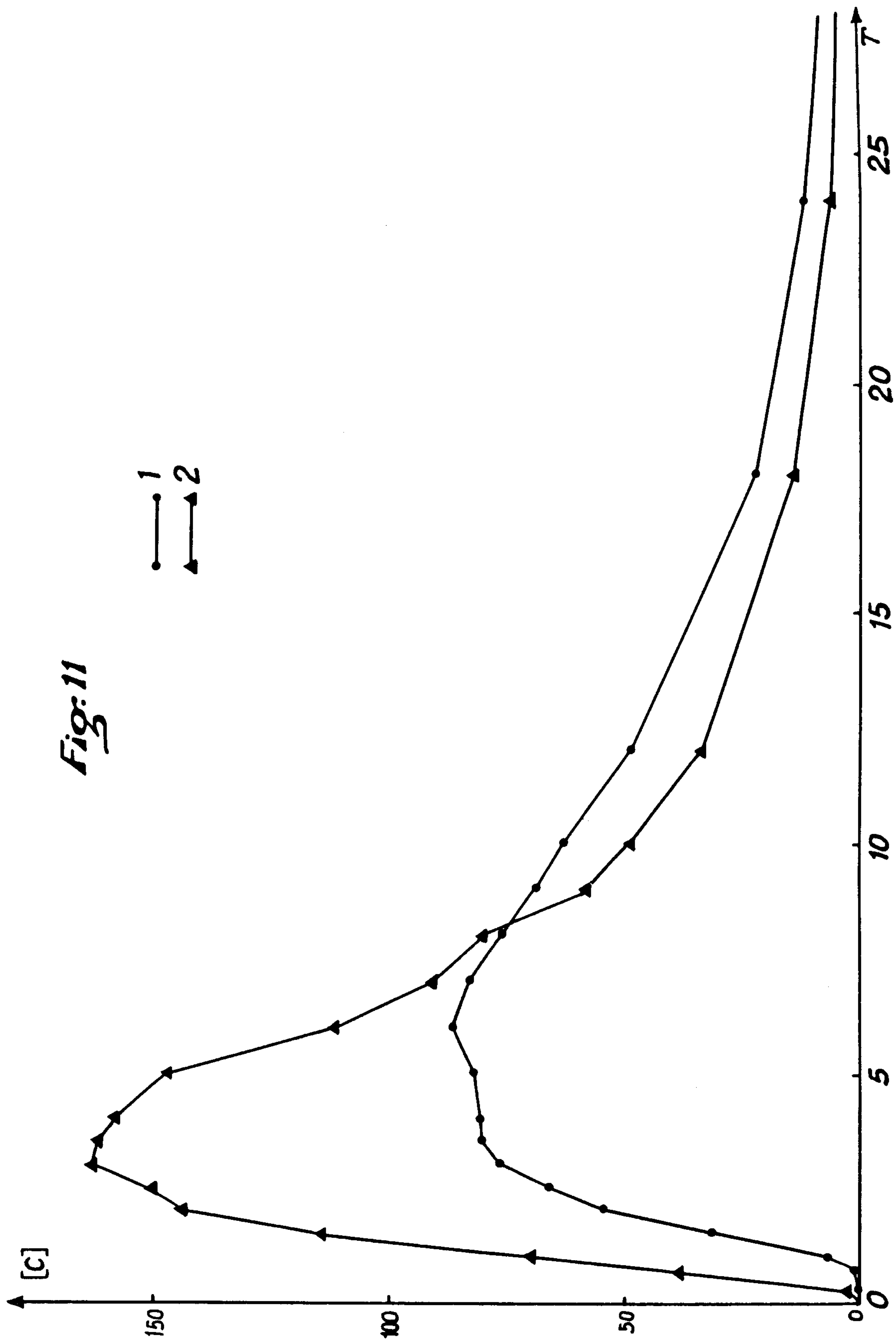
Fig:11
[C]
150
100
50
0
0
5
10
15
20
25
T
1
2